# FASCICULE DE BREVET EUROPEEN

(11) **EP 1 182 187 B1**
(45) Date de publication et mention de la délivrance du brevet: **06.10.2004**
(21) Numéro de dépôt: 01401919.4
(22) Date de dépôt: 18.07.2001
(51) Int. Cl.: C07C 45/50

(54) **Procédé améloiré d'hydroformylation mettant en oeuvre un catalyseur à base de cobalt et/ou de rhodium dans un solvant ionique non aqueux**
Verbessertes Hydroformylierungsverfahren mittels eines Katalysators auf Cobalt und/oder Rhodium-Basis in einem nichtwässrigen ionischen Lösungsmittel
Improved hydroformylation process with a catalyst based on cobalt and/or rhodium in a nonaqueous ionic solvent

(30) Priorité: 23.08.2000 FR 0010971
(43) Date de publication de la demande: 27.02.2002
(73) Titulaire: Institut Français du Pétrole, 92852 Rueil-Malmaison Cedex (FR)
(72) Inventeur: Favre, Frédéric, 69190 Saint Fons (FR); Commereuc, Dominique, 92190 Meudon (FR); Olivier-Bourbigou, Hélène, Rueil-Malmaison (FR); Saussine, Lucien, 78290 Croissy sur Seine (FR)

(56) Documents cités:
- EP-A- 0 157 316
- EP-A- 0 602 463
- EP-A- 0 776 880
- EP-A- 0 924 182
- DE-A- 19 919 494
- WASSERSCHEID, PETER ET AL: "Cationic phosphine ligands with phenylguanidinium modified xanthene moieties-a successful concept for highly regioselective, biphasic hydroformylation of oct-1-ene in hexafluorophosphate ionic liquids" CHEM. COMMUN. (CAMBRIDGE, U. K.) (2001), (5), 451-452 , XP002182178
- OLIVIER, HELENE ET AL: "Nonaqueous room-temperature ionic liquids: a new class of solvents for catalytic organic reactions" CHEM. IND. (DEKKER) (1996), 68(CATALYSIS OF ORGANIC REACTIONS), 249-263, XP000938205

## Description

La présente invention concerne un procédé amélioré d'hydroformylation de composés oléfiniquement insaturés au moyen d'un catalyseur à base de cobalt et/ou de rhodium mis en oeuvre en milieu biphasique. L'une des phases est constituée par un solvant ionique non-aqueux comprenant au moins un cation ammonium et/ou phosphonium quaternaire Q⁺ et au moins un anion A⁻. Le catalyseur comprend au moins un complexe du cobalt et/ou du rhodium coordiné par au moins un ligand choisi dans le groupe formé par les ligands azotés ou phosphorés portant en outre une fonction ionique (Q')⁺(A')⁻ telle que Q et Q' et/ou A et A' sont de nature chimique identique.

L'hydroformylation des composés oléfiniques est une réaction de grande importance industrielle et la plupart des procédés ont recours à des catalyseurs homogènes dissous dans une phase organique constituée des réactifs, des produits et éventuellement d'un excès de ligand, si bien que l'on rencontre des difficultés pour séparer et récupérer le catalyseur, en particulier quand celui-ci est employé en relativement grande quantité, comme c'est le cas avec les catalyseurs à base de cobalt, ou quand celui-ci est un métal noble, comme c'est le cas avec les catalyseurs à base de rhodium.

Une solution en vue de résoudre ce problème a été évoquée par Bartik et al. : Organometallics (1993) 12 164-170, J. Organometal. Chem. (1994) 480 15-21, ainsi que par Beller et al. : J. Molecular Catal. A: Chemical (1999) 143 31-39. Elle consiste à effectuer l'hydroformylation en présence d'une solution aqueuse contenant un complexe du cobalt qui est rendu hydrosoluble grâce à la présence d'un ligand phosphine-sulfonate, tel que le sel de sodium de la triphénylphosphine trisulfonée ou d'une tris-(alkylphényl)-phosphine trisulfonée. La demande internationale WO-A-97/00 132 décrit des clusters de cobalt substitués par des groupements trialkoxysilylméthyle qui les rendent solubles dans l'eau. De cette manière, la phase organique contenant les aldéhydes est aisément séparée de la phase aqueuse contenant le catalyseur.

Une autre solution en vue de résoudre ce problème a été décrite dans le brevet français FR-B-2 314 910. Elle consiste à effectuer l'hydroformylation en présence d'une solution aqueuse contenant un complexe du rhodium qui est rendu hydrosoluble grâce à la présence d'un ligand phosphine sulfonée lui-même hydrosoluble, tel que le sel de sodium de la triphénylphosphine trisulfonée. De cette manière, la phase organique contenant les aldéhydes est aisément séparée de la phase aqueuse contenant le catalyseur. Cette technique a fait l'objet d'un nombre considérable de travaux qui ont été discutés dans un article de W.A. Herrmann paru dans Angewandte Chemie International en 1993, volume 32, page 1524 et suivantes.

En dépit du grand intérêt industriel de ces techniques pour l'hydroformylation du propylène, ces systèmes à deux phases souffrent du manque de solubilité des oléfines dans l'eau, ce qui conduit à des vitesses de réaction relativement faibles qui les rendent inapplicables pour les oléfines à longue chaîne.

Par ailleurs, a été décrit dans le brevet US-A-3 565 823 une technique consistant à disperser un composé d'un métal de transition dans un sel d'étain ou de germanium et d'un ammonium ou d'un phosphonium quaternaire, de formule (R¹R²R³R⁴Z)YX₃ dans laquelle R¹, R², R³ et R⁴, sont des restes hydrocarbyles ayant jusqu'à 18 atomes de carbone, Z est l'azote ou le phosphore, Y l'étain ou le germanium et X un halogène, par exemple le chlore ou le brome. Dans le brevet US-A-3 832 391 a été décrit un procédé de carbonylation des oléfines utilisant une telle composition. Les compositions précédentes présentent le désavantage d'avoir un point de fusion relativement élevé, par exemple supérieur à 90 °C, ce qui complique la manipulation des solutions de catalyseur et des produits de réaction.

Il a été décrit dans le brevet US-A-5 874 638 de la demanderesse que l'on peut à la fois bénéficier des avantages d'une mise en oeuvre à deux phases tout en évitant les inconvénients liés d'une part à l'utilisation de l'eau et d'autre part à l'emploi de composés à point de fusion élevé, en dissolvant certains composés catalytiques des métaux de transition des groupes 8, 9 et 10, connus pour catalyser l'hydroformylation, dans des solvants ioniques non aqueux qui sont constitués par des sels organiques-inorganiques liquides à température ambiante.

Il a maintenant été trouvé que, dans la réaction d'hydroformylation catalysée par des complexes à base de cobalt et/ou de rhodium mis en oeuvre dans un solvant ionique non-aqueux comprenant au moins un cation ammonium et/ou phosphonium quaternaire Q⁺ et au moins un anion A⁻, liquide à une température inférieure à 90 °C, le taux de rétention du métal dans le solvant ionique est grandement amélioré lorsque le catalyseur comprend au moins un complexe du cobalt et/ou du rhodium coordiné par au moins un ligand choisi dans le groupe formé par les ligands azotés ou phosphorés portant en outre une fonction ionique (Q')⁺(A')⁻ telle que Q et Q' et/ou A et A' sont de nature chimique identique.

Plus précisément. l'invention a pour objet un procédé pour l'hydroformylation en phase liquide des composés oléfiniquement insaturés dans lequel la réaction est effectuée en présence d'un solvant consistant en un liquide ionique non-aqueux comprenant au moins un sel de formule générale Q⁺A⁻, dans laquelle Q⁺ représente un ammonium quaternaire et/ou un phosphonium quaternaire, et A- représente un anion, et d'au moins un complexe du cobalt et/ou du rhodium coordiné par au moins un ligand choisi dans le groupe formé par les ligands azotés ou phosphorés portant en outre une fonction ionique (Q')⁺(A')⁻ telle que au moins le cation (Q')⁺ ou l'anion (A')⁻ aient la même nature chimique que le cation Q⁺ ou l'anion A⁻ dudit liquide ionique non-aqueux.

Le solvant ionique non-aqueux est choisi dans le groupe formé par les sels liquides qui ont pour formule générale Q⁺ A⁻ dans laquelle Q⁺ représente un ammonium quaternaire et/ou un phosphonium quaternaire et A⁻ représente tout anion susceptible de former un sel liquide à basse température, c'est-à-dire en dessous de 90 °C, avantageusement d'au plus 85 °C, et de préférence au-dessous de 50 °C. Les anions A⁻ préférés sont les ions nitrate, sulfate, phosphate, acétate, halogéno-acétates, tétrafluoro-borate, tétrachoro-borate, hexafluoro-phosphate, hexafluoro-antimonate, fluoro-sulfonate, perfluoroalkyl-sulfonates, arène-sulfonates, ces derniers éventuellement substitués par des groupements halogéne ou halogénoalkyle.

Les ammonium et/ou phosphonium quaternaires Q⁺ répondent de préférence aux formules générales NR¹R²R³R⁴⁺ et PR¹R²R³R⁴⁺, ou aux formules générales R¹R²N=CR³R⁴⁺ et R¹R²P=CR³R⁴⁺, où R¹, R², R³ et R⁴, identiques ou différents, représentent l'hydrogène (à l'exception du cation NH₄⁺ pour NR¹R²R³R⁴⁺), de préférence un seul substituant représentant l'hydrogène, ou des restes hydrocarbyles ayant de 1 à 30 atomes de carbone, par exemple des groupements alkyles, saturés ou non saturés, cycloalkyles ou aromatiques, aryles ou aralkyles, éventuellement substitués, comprenant de 1 à 30 atomes de carbone. Les ammonium et/ou phosphonium peuvent également être dérivés d'hétérocycles azotés et/ou phosphorés comportant 1, 2 ou 3 atomes d'azote et/ou de phosphore, dans lesquelles les cycles sont constitués de 4 à 10 atomes, de préférence 5 à 6 atomes.

L'ammonium ou le phosphonium quaternaire peut également être un cation de formule :

R¹R²⁺N=CR³-R⁵-R³C=N⁺R¹R²

ou

R¹R²⁺P=CR³-R⁵-R³C=P⁺R¹R²

dans laquelle R¹, R² et R³, identiques ou différents, sont définis comme précédemment et R⁵ représente un reste alkylène ou phénylène.

Parmi les groupements R¹, R², R³ et R⁴ on mentionnera les radicaux méthyle, éthyle, propyle, isopropyle, butyle, secondaire butyle, tertiaire butyle, amyle, méthylène, éthylidène, phényle ou benzyle ; R⁵ pourra être un groupement méthylène, éthylène, propylène ou phénylène.

Le cation ammonium et/ou phosphonium Q⁺ est choisi de préférence dans le groupe formé par le N-butylpyridinium, le N-éthylpyridinium, le pyridinium, l'éthyl-3 méthyl-1 imidazolium, le butyl-3 méthyl-1 imidazolium, le diéthylpyrazolium, le N-butyl-N-méthylpyrrolidinium, le triméthylphényl-ammonium, le tétrabutylphosphonium, le tributyl-(tétradécyl)-phosphonium. A titre d'exemples des sels utilisables selon l'invention on peut citer l'hexafluorophosphate de N-butyl-pyridinium, le tétrafluoroborate de N-éthyl-pyridinium, le fluorosulfonate de pyridinium, le tétrafluoroborate de butyl-3 méthyl-1 imidazolium, l'hexafluoroantimonate de butyl-3 méthyl-1 imidazolium, l'hexafluorophosphate de butyl-3 méthyl-1 imidazolium, le trifluoroacétate de butyl-3 méthyl-1 imidazolium, le trifluorométhylsulfonate de butyl-3 méthyl-1 imidazolium, l'hexafluorophosphate de triméthyl-phénylammonium, le tétrafluoroborate de tétrabutylphosphonium. Ces sels peuvent être utilisés seuls ou en mélange.

Les composés du cobalt et/ou du rhodium précurseurs du catalyseur sont choisis dans le groupe formé par les sels de cobalt et/ou de rhodium, comme les acétylacétonates, les carboxylates et en particulier le formiate ou l'acétate, et les complexes carbonyles, comme le dicobalt-octacarbonyle, l'hydrure de cobalt-tétracarbonyle, l'acétylacétonate de rhodium-dicarbonyle et les clusters carbonyles. Le choix du composé précurseur du cobalt et/ou du rhodium n'est pas critique, mais on préfère en général éviter les halogénures.

Le ligand azoté est choisi dans le groupe formé par les mono-amines, les di-, tri- et polyamines, les imines, les di-imines, les pyridines, les bipyridines, les imidazoles, les pyrroles, les pyrazoles, tous comportant en outre dans leur formule au moins un substituant porteur d'une fonction ionique (Q')⁺(A')⁻ telle que au moins le cation (Q')⁺ ou l'anion (A')⁻ aient la même nature chimique que le cation Q⁺ ou l'anion A⁻ du solvant ionique non aqueux défini ci-dessus.

Le ligand phosphoré est choisi dans le groupe formé par les phosphines, les poly-phosphines, les oxydes de phosphines, les phosphites, tous comportant en outre dans leur formule au moins un substituant porteur d'une fonction ionique (Q')⁺(A')⁻ telle qu'au moins le cation (Q')⁺ ou l'anion (A')⁻ ait la même nature chimique que le cation Q⁺ ou l'anion A⁻ du solvant ionique non aqueux défini ci-dessus.

On peut citer à titre d'exemples non limitatifs les associations entre les ligands et les sels fondus suivants :
- les ligands 1-(4-pyridyl)-2-(dicyclopentyl-méthyl-phosphonium)-éthane tétrafluoroborate (1) et 1-(N-imidazolyl)-2-(dicyclopentyl-méthyl-phosphonium)-éthane tétrafluoroborate (2), mis en oeuvre notamment dans les solvants ioniques constitués par les tétrafluoroborates d'ammonium ou de phosphonium quaternaires et par les sels comprenant les cations dicyclopentyl-méthyl-alkyl-phosphonium ;
- le ligand 1-(diphénylphosphino)-2-(4-N-méthyl-pyridinium)-éthane hexafluorophosphate (3), mis en oeuvre notamment dans les solvants ioniques constitués par les hexafluorophosphates d'ammonium ou de phosphonium quaternaires et par les sels comprenant les cations 4-alkyl-N-méthyl-pyridinium, le ligand 1-(dicyclopentylphosphino)-2-(3-méthyl-1-imidazolium)-éthane hexafluorophosphate (4), mis en oeuvre notamment dans les solvants ioniques constitués par les hexafluorophosphates d'ammonium ou de phosphonium quaternaires et par les sels comprenant les cations alkyl-3-méthyl-1-imidazolium, 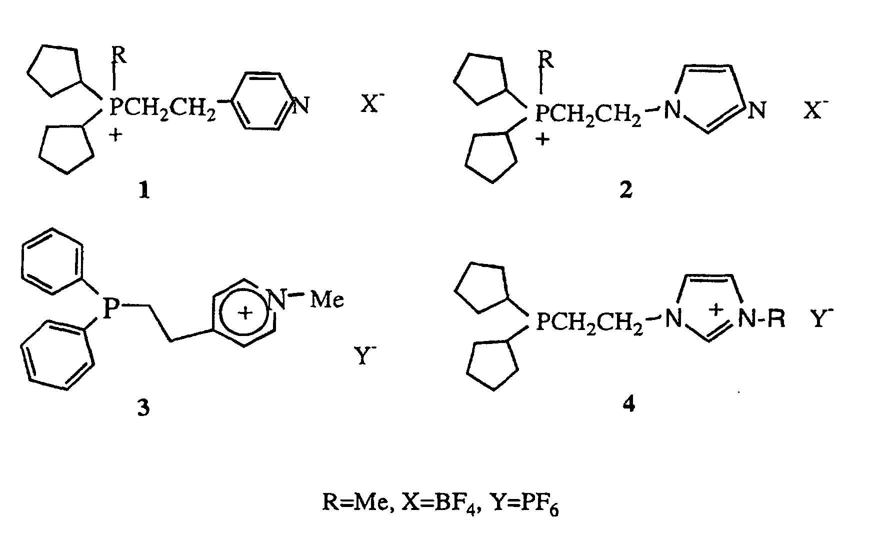
- le ligand N-(3-diphénylphosphinophényl)-N'-diméthyl-guanidinium tétrafluoroborate (5), mis en oeuvre notamment dans les solvants ioniques constitués par les tétrafluoroborates d'ammonium ou de phosphonium quaternaires et par les sels comprenant les cations N-phényl-N'-dialkyl-guanidinium ;
- le ligand tris-(phényl-3-sulfonate de tétrabutylammonium)-phosphine (triphénylphosphine trisulfonate de tétrabutylammonium (6), mis en oeuvre notamment dans les solvants ioniques constitués par les sels de tétrabutylammonium et par les sels comprenant les anions sulfonate, comme par exemple les tosylates et les triflates ;
- le ligand tris-(phényl-3-sulfonate de sodium)-phosphine ((triphénylphosphine trisulfonate de sodium) (7), mis en oeuvre notamment dans les solvants ioniques constitués par les sels comprenant les anions sulfonate, comme par exemple les tosylates et les triflates ; 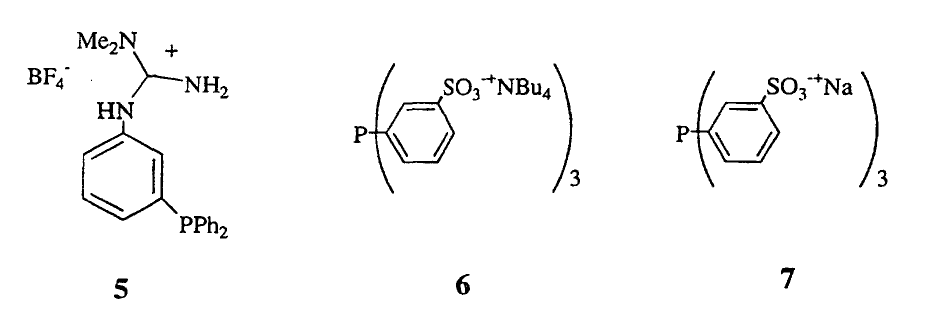
- et le ligand (di-t-butyl-3,5-catecholato)-(phénoxy-4-sulfonate de tétrabutylammonium)-phosphite (8), mis en oeuvre notamment dans les solvants ioniques constitués par les sels de tétrabutylammonium et par les sels comprenant les anions sulfonate, comme par exemple les tosylates et les triflates.

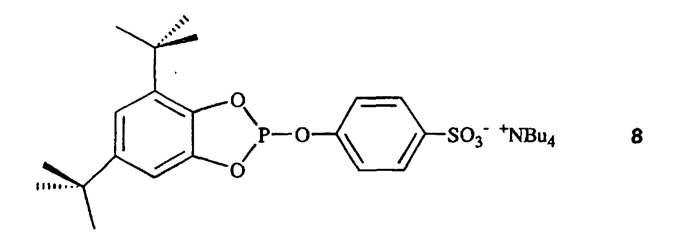

La composition catalytique est obtenue par mélange, d'une manière quelconque, du sel liquide avec le composé du cobalt et/ou du rhodium et le ligand. On peut également dissoudre préalablement le composé du métal de transition et/ou le ligand dans un solvant organique.

Le complexe entre le précurseur de cobalt et/ou du rhodium et le ligand peut être préparé préalablement à la réaction par mélange du précurseur de cobalt et/ou du rhodium avec le ligand dans un solvant convenable, par exemple un solvant organique ou le solvant ionique non aqueux qui sera utilisé ensuite dans la réaction catalytique. Le complexe peut aussi être préparé in situ par mélange du précurseur de cobalt et/ou du rhodium et du ligand directement dans le réacteur d'hydroformylation.

La concentration du complexe de cobalt et/ou de rhodium dans le solvant ionique liquide n'est pas critique. Elle est avantageusement comprise entre 0,1 mmole et 5 moles par litre de solvant ionique liquide, de préférence entre 1 mmole et 1 mole par litre, et de manière encore plus préférée entre 10 et 500 mmoles par litre. Le rapport molaire entre le ligand et le composé du cobalt et/ou du rhodium est compris entre 0,1 et 500, de préférence entre 1 et 100.

Les composants entrant dans la composition selon l'invention peuvent être mélangés dans un ordre quelconque, à une température comprise entre -20 °C et 200 °C, de préférence entre 0 °C et 140 °C et avantageusement entre 20 °C et 90 °C.

Les composés oléfiniquement insaturés susceptibles d'être hydroformylés sont choisis dans le groupe formé par les monooléfines, les dioléfines, en particulier les dioléfines conjuguées, les composés oléfiniques comportant un ou plusieurs hétéroatomes, notamment dans des groupements insaturés comme les fonctions cétone et acide carboxylique. A titre d'exemples on peut citer l'hydroformylation des pentènes en hexanal et méthylpentanal, des hexènes en isoheptanals, des isooctènes en isononanals et des coupes C₁₀ à C₁₆ oléfiniques en aldéhydes C₁₁ à C₁₇. Ces composés oléfiniques peuvent être mis en oeuvre purs ou dilués par des hydrocarbures saturés ou d'autres hydrocarbures insaturés.

Le rapport des pressions partielles de l'hydrogène et du monoxyde de carbone utilisé dans le milieu réactionnel pour l'hydroformylation peut être de 10:1 à 1:10, de préférence dans un rapport 1:1, mais tout autre rapport peut être utilisé selon la mise en oeuvre du procédé.

La température à laquelle se fera l'hydroformylation sera comprise entre 30 °C et 200 °C, avantageusement la température est inférieure à 150 °C, de préférence comprise entre 50 °C et moins de 150°C. La pression peut être comprise entre 1 MPa et 20 MPa, de préférence entre 2 MPa et 15 MPa.

La réaction catalytique d'hydroformylation des composés insaturés peut être conduite en système fermé, en système semi-ouvert ou en continu avec un ou plusieurs étages de réaction. A la sortie du réacteur la phase organique contenant les produits de réaction est séparée avantageusement par simple décantation de la phase solvant ionique contenant le "sel fondu" et la majeure partie du catalyseur. Cette phase solvant ionique qui contient au moins en partie le catalyseur, est, au moins en partie, retournée au réacteur, l'autre partie étant traitée pour éliminer les résidus du catalyseur.

Les exemples suivants illustrent l'invention sans en limiter la portée.

### EXEMPLE 1

La réaction d'hydroformylation est conduite dans un autoclave en acier inoxydable d'une contenance de 100 mL, muni d'une double enveloppe permettant la régulation de la température par circulation d'un fluide caloporteur. Dans cet autoclave purgé au préalable de l'air et de l'humidité et placé sous pression atmosphérique du mélange hydrogène-monoxyde de carbone (1/1 molaire), on introduit 0,0193 g d'acétylacétonate de rhodium-dicarbonyle (soit 0,075 mmole de rhodium), 4 équivalents molaires de triphénylphosphine-trisulfonate de sodium, 4 mL de trifluorométhylsulfonate de butyl-3-méthyl-1-imidazolium, 2 mL d'heptane (standard) et 7,5 mL d'hexène-1. Dans cet exemple, l'anion sulfonate est l'ion commun au ligand et au solvant ionique non aqueux. On porte la pression du mélange hydrogène-monoxyde de carbone (1/1 molaire) à 2 MPa et la température à 80 °C et on met l'agitation en route. Après 2 heures on arrête l'agitation et on laisse refroidir et décanter le mélange réactionnel, puis on relache la pression. Après soutirage hors de l'autoclave, la phase organique supérieure est incolore. La conversion de l'hexène-1 est de 99 % en poids. La sélectivité pour les aldéhydes en C7 est de 93 % et le rapport n/iso (n-heptanal / isoheptanals) est égal à 3,5. L'analyse de la phase organique supérieure montre qu'elle contient moins de 5 ppm de rhodium métal (ppm : parties par million en poids).

### EXEMPLE 2 (comparatif)

La réaction d'hydroformylation est conduite dans le même appareillage et selon le même mode opératoire que décrit dans l'exemple 1. Dans cet autoclave purgé au préalable de l'air et de l'humidité et placé sous pression atmosphérique du mélange hydrogène-monoxyde de carbone (1/1 molaire), on introduit 0,0193 g d'acétylacétonate de rhodium-dicarbonyle (soit 0,075 mmole de rhodium), 4 équivalents molaires de triphénylphosphine-trisulfonate de sodium, 4 mL d'hexafluorophosphate de butyl-3-méthyl-1-imidazolium, 2 mL d'heptane (standard) et 7,5 mL d'hexène-1. Dans cet exemple comparatif, il n'y a pas d'ion en commun entre le ligand et le solvant ionique non aqueux. On porte la pression du mélange hydrogène-monoxyde de carbone (1/1 molaire) à 2 MPa et la température à 80 °C et on met l'agitation en route. Après 3 heures on arrête l'agitation et on laisse refroidir et décanter le mélange réactionnel, puis on relâche la pression. Après soutirage hors de l'autoclave, la phase organique supérieure est incolore. La conversion de l'hexène-1 est de 74 % en poids. La sélectivité pour les aldéhydes en C7 est de 48 % et le rapport n/iso (n-heptanal / isoheptanals) est égal à 2,7. L'analyse de la phase organique supérieure montre qu'elle contient 195 ppm de rhodium métal (ppm : parties par million en poids).

### EXEMPLE 3

La réaction d'hydroformylation est conduite dans le même appareillage et selon le même mode opératoire que décrit dans l'exemple 1. On introduit 0,0193 g d'acétylacétonate de rhodium-dicarbonyle (soit 0,075 mmole de rhodium), 4 équivalents molaires de triphénylphosphine-disulfonate de sodium, 4 mL de trifluorométhanesulfonate de butyl-3-méthyl-1-imidazolium, 2 mL d'heptane (standard) et 7,5 mL d'hexène-1. Dans cet exemple, l'anion sulfonate est l'ion commun au ligand et au solvant ionique non aqueux. On porte la pression du mélange hydrogène-monoxyde de carbone (1/1 molaire) à 2 MPa et la température à 80 °C et on met l'agitation en route. Après 2 heures on arrête l'agitation et on laisse refroidir et décanter le mélange réactionnel, puis on relâche la pression. Après soutirage hors de l'autoclave, la phase organique supérieure est incolore. La conversion de l'hexène-1 est de 98 % en poids. La sélectivité pour les aldéhydes en C7 est de 96 % et le rapport n/iso (n-heptanal / isoheptanals) est égal à 3,5. L'analyse de la phase organique supérieure montre qu'elle contient moins de 5 ppm de rhodium métal (ppm : parties par million en poids).

### EXEMPLE 4

La réaction d'hydroformylation est conduite dans le même appareillage et selon le même mode opératoire que décrit dans l'exemple 1. On introduit 0,0193 g d'acétylacétonate de rhodium-dicarbonyle (soit 0,075 mmole de rhodium), 10 équivalents molaires de N-(3-diphénylphosphinophényl)-N'-diméthylguanidinium tétrafluoroborate, 4 mL de tétrafluoroborate de butyl-3-méthyl-1-imidazolium, 2 mL d'heptane (standard) et 7,5 mL d'hexène-1. Dans cet exemple, l'anion tétrafluoroborate est l'ion commun au ligand et au solvant ionique non aqueux. On porte la pression du mélange hydrogène-monoxyde de carbone (1/1 molaire) à 2 MPa et la température à 80 °C et on met l'agitation en route. Après 2 heures on arrête l'agitation et on laisse refroidir et décanter le mélange réactionnel, puis on relâche la pression. Après soutirage hors de l'autoclave, la phase organique supérieure est incolore. La conversion de l'hexène-1 est de 77 % en poids. La sélectivité pour les aldéhydes en C7 est de 74 % et le rapport n/iso (n-heptanal / isoheptanals) est égal à 3. L'analyse de la phase organique supérieure montre qu'elle contient moins de 5 ppm de rhodium métal (ppm : parties par million en poids).

### EXEMPLE 5

La réaction d'hydroformylation est conduite dans le même appareillage et selon le même mode opératoire que décrit dans l'exemple 1. On introduit 0,0193 g d'acétylacétonate de rhodium-dicarbonyle (soit 0,075 mmole de rhodium), 7 équivalents molaires de 1-(diphénylphosphino)-2-(4-N-méthylpyridinium)-éthane tétrafluoroborate, 4 mL de tétrafluoroborate de butyl-3-méthyl-1-imidazolium, 2 mL d'heptane (standard) et 7,5 mL d'hexène-1. Dans cet exemple, l'anion tétrafluoroborate est l'ion commun au ligand et au solvant ionique non aqueux. On porte la pression du mélange hydrogène-monoxyde de carbone (1/1 molaire) à 2 MPa et la température à 80 °C et on met l'agitation en route. Après 4 heures on arrête l'agitation et on laisse refroidir et décanter le mélange réactionnel, puis on relâche la pression. Après soutirage hors de l'autoclave, la phase organique supérieure est incolore. La conversion de l'hexène-1 est de 84 % en poids. La sélectivité pour les aldéhydes en C7 est de 99 % et le rapport n/iso (n-heptanal / isoheptanals) est égal à 2,6. L'analyse de la phase organique supérieure montre qu'elle contient moins de 10 ppm de rhodium métal (ppm : parties par million en poids).

### EXEMPLE 6

La réaction d'hydroformylation est conduite dans un autoclave en acier inoxydable d'une contenance de 300 mL, muni d'une double enveloppe permettant la régulation de la température par circulation d'un fluide caloporteur, et équipé d'une agitation mécanique efficace avec pales et contre-pales. Dans cet autoclave purgé au préalable de l'air et de l'humidité et placé sous la pression atmosphérique du mélange hydrogène-monoxyde de carbone (1/1 molaire), on introduit 0,4 g de dicobalt-octacarbonyle (soit 2,3 mmole de cobalt), 1 équivalent molaire de 1-(4-pyridyl)-2-(dicyclopentyl-méthyl-phosphonium)-éthane tétrafluoroborate, 10 mL de tétrafluoro-borate de butyl-3-méthyl-1-imidazolium, 30 mL d'heptane et 30 mL d'hexène-1. On porte la pression du mélange hydrogène-monoxyde de carbone (1/1 molaire) à 9 MPa et la température à 95 °C et on met l'agitation en route. Après 6 heures on arrête l'agitation et on laisse refroidir et décanter le mélange réactionnel, puis on relâche la pression. Après soutirage hors de l'autoclave, la phase organique supérieure est légèrement colorée, indiquant que seulement des traces de cobalt ont été extraites. La conversion de l'hexène-1 est de 80 % en poids. La sélectivité pour les aldéhydes en C7 est de 96,4 % et le rapport n/iso (n-heptanal / isoheptanals) est égal à 3,6.

## Revendications

1. Procédé pour l'hydroformylation en phase liquide des composés oléfiniquement insaturés dans lequel la réaction est effectuée en présence d'un solvant consistant en un liquide ionique non-aqueux comprenant au moins un sel de formule générale Q⁺A⁻, dans laquelle Q⁺ représente un ammonium quaternaire et/ou un phosphonium quaternaire, et A- représente un anion, et d'au moins un complexe du cobalt et/ou du rhodium coordiné par au moins un ligand choisi dans le groupe formé par les ligands azotés ou phosphorés portant en outre une fonction ionique (Q')⁺(A')⁻ telle que au moins le cation (Q')⁺ ou l'anion (A')⁻ aient la même nature chimique que le cation Q⁺ ou l'anion A⁻ dudit liquide ionique non-aqueux.

2. Procédé selon la revendication 1, **caractérisé en ce que** ledit liquide ionique non-aqueux est choisi dans le groupe formé par les sels liquides de formule générale Q⁺ A⁻ dans laquelle Q⁺ représente un ammonium quaternaire et/ou un phosphonium quaternaire et A⁻ représente tout anion susceptible de former un sel liquide à basse température, c'est-à-dire en dessous de 90 °C.

3. Procédé selon la revendication 1 ou 2, **caractérisé en ce que** les anions A⁻ sont choisis parmi les ions nitrate, sulfate, phosphate, acétate, halogéno-acétates, tétrafluoro-borate, tétrachoro-borate, hexafluoro-phosphate, hexafluoro-antimonate, fluoro-sulfonate, perfluoroalkyl-sulfonates, arène-sulfonates, ces derniers étant éventuellement substitués par des groupements halogène ou halogénoalkyle.

4. Procédé selon l'une des revendications 1 à 3, **caractérisé en ce que** les ammonium et/ou phosphonium quaternaires répondent aux formules générales NR¹R²R³R⁴⁺ et PR¹R²R³R⁴⁺, ou aux formules générales R¹R²N=CR³R⁴⁺ et R¹R²P=CR³R⁴⁺ où R¹, R², R³ et R⁴, identiques ou différents, représentent l'hydrogène, à l'exception du cation NH₄⁺, un seul substituant représentant l'hydrogène, ou des restes hydrocarbyles ayant de 1 à 30 atomes de carbone.

5. Procédé selon l'une des revendications 1 à 3, **caractérisé en ce que** les ammonium et/ou phosphonium sont dérivés d'hétérocycles azotés et/ou phosphorés comportant 1, 2 ou 3 atomes d'azote et/ou de phosphore, dans lesquelles les cycles sont constitués de 4 à 10 atomes, de préférence 5 à 6 atomes.

6. Procédé selon l'une des revendications 1 à 3, **caractérisé en ce que** l'ammonium ou le phosphonium quaternaire sont constitués par un cation de formule :
R¹R²⁺N=CR³-R⁵-R³C=N⁺R¹R²
ou
R¹R²⁺P=CR³-R⁵-R³C=P⁺R¹R²
dans laquelle R¹, R² et R³, identiques ou différents, sont définis comme précédemment et R⁵ représente un reste alkylène ou phénylène.

7. Procédé selon l'une des revendications 1 à 6, **caractérisé en ce que** les ammonium et/ou phosphonium sont choisis dans le groupe formé par le N-butylpyridinium, le N-éthylpyridinium, le pyridinium, l'éthyl-3 méthyl-1 imidazolium, le butyl-3 méthyl-1 imidazolium, le diéthylpyrazolium, le N-butyl-N-méthylpyrrolidinium, le triméthylphénylammonium, le tétrabutylphosphonium, le tributyl-(tétradécyl)-phosphonium.

8. Procédé selon l'une des revendications 1 à 7, **caractérisé en ce que** le solvant ionique non-aqueux est choisi dans le groupe formé par l'hexafluorophosphate de N-butyl-pyridinium, le tétrafluoroborate de N-éthyl-pyridinium, le fluorosulfonate de pyridinium, le tétrafluoroborate de butyl-3 méthyl-1 imidazolium, l'hexafluoro-antimonate de butyl-3 méthyl-1 imidazolium, l'hexafluorophosphate de butyl-3 méthyl-1 imidazolium, le trifluoroacétate de butyl-3 méthyl-1 imidazolium, le trifluorométhylsulfonate de butyl-3 méthyl-1 imidazolium, l'hexafluorophosphate de triméthyl-phénylammonium, le tétrafluoroborate de tétrabutylphosphonium.

9. Procédé selon l'une des revendications 1 à 8, **caractérisé en ce que** les composés du cobalt et/ou du rhodium précurseurs du catalyseur sont choisis dans le groupe formé par les sels de cobalt et/ou du rhodium et les complexes carbonyles.

10. Procédé selon l'une des revendications 1 à 9, **caractérisé en ce que** les composés du cobalt et/ou du rhodium précurseurs du catalyseur sont choisis dans le groupe formé par les acétylacétonates, les carboxylates, le dicobalt-octacarbonyle, l'hydrure de cobalt-tétracarbonyle, l'acétylacétonate de rhodium-dicarbonyle et les clusters carbonyles.

11. Procédé selon l'une des revendications 1 à 10, **caractérisé en ce que** le ligand azoté est choisi dans le groupe formé par les mono-amines, les di-, tri- et polyamines, les imines, les di-imines, les pyridines, les bipyridines, les imidazoles, les pyrroles, les pyrazoles, tous comportant en outre dans leur formule au moins un substituant porteur d'une fonction ionique (Q')⁺(A')⁻ telle que au moins le cation (Q')⁺ ou l'anion (A')⁻ aient la même nature chimique que le cation Q⁺ ou l'anion A⁻ du solvant ionique non aqueux défini ci-dessus.

12. Procédé selon l'une des revendications 1 à 10, **caractérisé en ce que** le ligand phosphoré est choisi dans le groupe formé par les phosphines, les polyphosphines, les oxydes de phosphines, les phosphites, tous comportant en outre dans leur formule au moins un substituant porteur d'une fonction ionique (Q')⁺(A') telle que au moins le cation (Q')⁺ ou l'anion (A')⁻ aient la même nature chimique que le cation Q⁺ ou l'anion A⁻ du solvant ionique non aqueux défini ci-dessus.

13. Procédé selon l'une des revendications 1 à 12, **caractérisé en ce que** la concentration du complexe de cobalt et/ou de rhodium dans le solvant ionique liquide est comprise entre 0,1 mmoles par litre et 5 moles par litre et le rapport molaire entre le ligand azoté ou le ligand phosphoré et le composé du cobalt et/ou du rhodium est compris entre 0,1 et 500.

14. Procédé selon l'une des revendications 1 à 13, **caractérisé en ce que** l'on soumet à la réaction d'hydroformylation au moins un composé oléfiniquement insaturé choisi dans le groupe formé par les monooléfines, les dioléfines et en particulier les dioléfines conjuguées, les composés oléfiniques comportant un ou plusieurs hétéroatomes, notamment dans des groupements insaturés comme les fonctions cétone et acide carboxylique.

15. Procédé selon l'une des revendications 1 à 14, **caractérisé en ce que** la réaction d'hydroformylation est effectuée avec un rapport des pressions partielles de l'hydrogène et du monoxyde de carbone de 10:1 à 1:10, à une température comprise entre 30 °C et 200 °C, sous une pression comprise entre 1 MPa et 20 MPa.

## Patentansprüche

1. Verfahren zur Hydroformylierung von olefinisch ungesättigten Verbindungen in flüssiger Phase, bei welchem die Reaktion in Gegenwart eines Lösungsmittels durchgeführt wird, welches aus einer nichtwäßrigen ionischen Lösung besteht, und umfassend zumindest ein Salz der allgemeinen Formel Q⁺A⁻, worin Q⁺ ein quarternäres Ammonium und/oder quarternäres Phosponium darstellt, und A-ein Anion darstellt, und mindestens einen Kobalt- und/oder Rhodiumkomplex, welcher koordiniert wird mittels mindestens eines Liganden ausgewählt aus der Gruppe gebildet aus stickstoff- oder phosphorhaltigen Liganden, welche außerdem eine ionische (Q')⁺(A')⁻ Funktion aufweisen, in der Art, dass mindestens das Kation (Q')⁺ oder das Anion (A')⁻ dieselbe chemische Eigenschaft haben, wie das Kation Q⁺ oder das Anion A⁻ der besagten nichtwäßrigen ionischen Flüssigkeit.

2. Verfahren nach dem Anspruch 1, **dadurch gekennzeichnet, dass** die besagte nichtwäßrige ionische Flüssigkeit ausgewählt ist aus der Gruppe gebildet aus den flüssigen Salzen der allgemeinen Formel Q⁺ A⁻, worin Q⁺ ein quarternäres Ammonium und/oder ein quarternäres Phosphonium darstellt und A⁻ jedes Anion darstellt, welches geeignet ist, ein flüssiges Salz bei niedriger Temperatur, also unterhalb von 90° C, zu bilden.

3. Verfahren nach Anspruch 1 oder 2, **dadurch gekennzeichnet, dass** die A⁻ Anionen ausgewählt sind aus den Nitrat-, Sulfat-, Phosphat-, Azetat-, Halogenazetat-, Tetrafluoroborat-, Tetrachloroborat-, Hexafluorophosphat-, Hexafluoroantimonat-, Fluorosulfonat-, Perfluoroalkylsulfonat-, Arensulfonat-Ionen, wobei letztere durch Halogen- oder Halogenalkylgruppen substituiert sein können.

4. Verfahren nach einem der Ansprüche 1 bis 3, **dadurch gekennzeichnet, dass** das quaternäre Ammonium und/oder quarternäre Phosphonium den allgemeinen Formeln NR¹R²R³R⁴+ und PR¹R²R³R⁴+ entspricht, oder den allgemeinen Formeln R¹R²N=CR³R⁴+ und R¹R²P=CR³R⁴+ worin R¹, R², R³ und R⁴, identisch oder unterschiedlich, Wasserstoff darstellen, mit Ausnahme des Kations NH₄⁺, wobei ein einziger Substituent Wasserstoff oder ein Kohlenwasserstoffrest mit 1 bis 30 Kohlenstoffatomen darstellt.

5. Verfahren nach einem der Ansprüche 1 bis 3, **dadurch gekennzeichnet, dass** die Ammonium- und/oder die Phosphonium-Verbindungen Derivate stickstoffund/oder phosphorhaltiger Heterozyklen sind, umfassend 1, 2 oder 3 Stickstoff- und/oder Phosphoratome, in denen die Ringe aus vier bis zehn Atomen aufgebaut sind, vorzugsweise aus 5 bis 6 Atomen.

6. Verfahren nach einem der Ansprüche 1 bis 3, **dadurch gekennzeichnet, dass** das quarternäre Ammonium oder Phosphonium aus einem Kation aufgebaut sind mit der Formel:
R¹R²+N=CR³-R⁵-R³C=N+R¹R²
oder
R¹R²+P=CR³-R⁵-R³C=P+R¹R²
worin R¹, R² und R³, identisch oder unterschiedlich, wie vorhergehend definiert sind und R⁵ einen Alkylen- oder Phenylenrest darstellt.

7. Verfahren nach einem der Ansprüche 1 bis 6, **dadurch gekennzeichnet, dass** die Ammonium- und/oder Phosphonium-Verbindung ausgewählt ist aus der Gruppe gebildet aus N-Butylpyridinioum, N-Ethylpyridinium, Pyridinium, 1-Methyl-3-Ethyl-Imidazolium, 1-Methyl-3-Butyl-Imidazolium, Diethylpyrazolium, N-Butyl-N-methylpyrrolidinium, Trimethylphenylammonium, Tetrabutylphosphonium, Tributyl-(tetradecyl)-Phosphonium.

8. Verfahren nach einem der Ansprüche 1 bis 7, **dadurch gekennzeichnet, dass** das nicht-wässrige ionische Lösungsmittel ausgewählt ist aus der Gruppe gebildet aus N-Butyl-Pyridinium, dem Tetrafluoroborat von N-Ethyl-Pyridinium, dem Fluorosulfonat von Pyridinium, dem Tetrafluorborat von 1-Methyl-3-Butyl-Imidazolium, dem Hexafluoroantimonat von 1-Methyl-3-Butyl-Imidazolium, Hexafluorophosphat von 1-Methyl-3-Butyl-Imidazolium, dem Trifluoroacetat von 1-Methyl-3-Butyl-Imidazolium, dem Trifluoromethylsulfonat von 1-Methyl-3-Butyl-Imidazolin, dem Hexafluorophosphat von Trimethyl-phenylammonium, Tetrafluoroborate von Tetrabutylphosphonium.

9. Verfahren nach einem der Ansprüche 1 bis 8, **dadurch gekennzeichnet, dass** die Kobalt- und/oder Rhodium Vorläufer-Verbindungen des Katalysators ausgewählt sind aus der Gruppe gebildet aus den Kobalt- und/oder Rhodiumsalzen und Carbonylkomplexen.

10. Verfahren nach einem der Ansprüche 1 bis 9, **dadurch gekennzeichnet, dass** die Kobalt- oder Rhodium Vorläuferverbindungen des Katalysators ausgewählt sind aus der Gruppe gebildet aus Ethylacetonaten, Carboxylaten, Dikobalt-Octacarbonyl, dem Hydrid von Kobalt-tetracabonyl, Acetylacetonaten von Rhodium-Dicarbonyl und von Carbonylclustern.

11. Verfahren nach einem der Ansprüche 1 bis 10, **dadurch gekennzeichnet, dass** der stickstoffhaltige Ligand ausgewählt ist aus der Gruppe gebildet aus den Monoaminen, den Di-, Tri-, und Polyaminen, den Iminen, den Di-Iminen, den Pyridinen, den Bipyridinen, den Imidazolen, den Pyrrolen, den Pyrazolen, wobei diese alle darüber hinaus in ihrer Formel mindestens einen Substituenten umfassen, welcher eine ionische (Q')⁺ (A')⁻ Funktion aufweist, derart, dass mindestens das Kation (Q')⁺ oder das Anion (A')⁻ die gleiche chemische Eigenschaft haben wie das Kation Q⁺ oder das Anion A⁻ des nichtwäßrigen ionischen Lösungsmittels, welches oben definiert ist.

12. Verfahren nach einem der Ansprüche 1 bis 10, **dadurch gekennzeichnet, dass** der phosphorhaltige Ligand ausgewählt aus der Gruppe gebildet aus den Phosphinen, den Polyphosphinen, den Oxiden der Phosphine, den Phosphiten, wobei diese alle außerdem in ihren Formeln mindestens einen Substituenten mit einer ionischen (Q')⁺ (A')⁻ Funktion aufweisen, derart, dass das Kation (Q')⁺ oder das Anion (A')⁻ die gleiche chemische Eigenschaft hat wie das Kation Q⁺ oder das Anion A⁻ des oben definierten nichtwäßrigen ionischen Lösungsmittels.

13. Verfahren nach einem der Ansprüche 1 bis 12, **dadurch gekennzeichnet, dass** die Konzentration des Kobalt- und/oder Rhodiumkomplexes in dem flüssigen ionischen Lösungsmittel in dem Intervall zwischen 0,1 mmol pro Liter und 5 Mol pro Liter enthalten ist und das Molverhältnis zwischen dem stickstoffhaltigen Liganden oder dem phosphorhaltigen Liganden und der Kobalt- und/oder Rhodiumverbindung in dem Intervall zwischen 0,1 und 500 liegt.

14. Verfahren nach einem der Ansprüche 1 bis 13, **dadurch gekennzeichnet, dass** man der Hydroformylierungsreaktion mindestens eine olefinisch ungesättigte Verbindung unterwirft, welche ausgewählt ist aus der Gruppe gebildet durch Mono-Olefine, Diolefine und insbesondere konjugierte Olefine, wobei die olefinhaltigen Verbindungen ein oder mehrere Heteroatome umfassen, insbesondere in ungesättigten Gruppen wie Ketonen und Carboxylsäurefunktionen.

15. Verfahren nach einem der Ansprüche 1 bis 14, **dadurch gekennzeichnet, dass** die Hydroformylationsreaktion durchgeführt wird mit einem Partialdruckverhältnis von Wasserstoff zu Kohlenmonoxid von 10:1 bis 1:10, bei einer Temperatur zwischen 30° C und 200° C, unter einem Druck zwischen 1MPa und 20 MPa.

## Claims

1. A process for liquid phase hydroformylation of olefinically unsaturated compounds in which the reaction is carried out in the presence of a solvent consisting in a non-aqueous ionic comprising at least one salt with general formula Q⁺ A⁻, where Q⁺ represents a quaternary ammonium and/or phosphonium, and A⁻ represents an anion, and at least one cobalt and/or rhodium complex co-ordinated by at least one ligand selected from the group formed by nitrogen-containing or phosphorus-containing ligands also carrying an ionic function (Q')⁺(A')⁻ such that at least the cation (Q')⁺ or anion (A')⁻ has the same chemical nature as the cation Q⁺ or anion A⁻ of said non-aqueous ionic solvent.

2. A process according to claim 1, **characterised in that** the non-aqueous ionic solvent is selected from the group formed by liquid salts with general formula Q⁺ A⁻ where Q⁺ represents a quaternary ammonium and/or phosphonium cation and A⁻ represents any anion which can form a liquid salt at low temperature, i.e., below 90ºC.

3. A process according to claim 1 or 2, **characterised in that** the A⁻ anions are selected from nitrate, sulphate, phosphate, acetate, halogenoacetates, tetrafluoroborate, tetrachloroborate, hexafluorophosphate, hexafluoroantimonate, fluorosulphonate, perfluoroalkylsulphonates and arene-sulphonates, the latter optionally being substituted by halogen or halogenoalkyl groups.

4. A process according to any one of claims 1 to 3, **characterised in that** the quaternary ammoniums and/or phosphoniums have the general formulae NR¹R²R³R⁴⁺ and PR¹R²R³R⁴⁺ or the general formulae R¹R²N=C R³R⁴⁺ and R¹R²P=C R³R⁴⁺ where R¹, R², R³ and R⁴, which may be identical or different, represent hydrogen with the exception of the NH₄⁺ cation, one single substituent representing hydrogen, or hydrocarbyl residues containing 1 to 30 carbon atoms

5. A process according to any one of claims 1 to 3, **characterised in that** the ammoniums and/or phosphoniums are derived from nitrogen-containing and/or phosphorus-containing heterocycles containing 1, 2 or 3 nitrogen and/or phosphorus atoms, in which the cycles are constituted by 4 to 10 atoms, preferably 5 or 6 atoms.

6. A process according to any one of claims 1 to 3, **characterised in that** the quaternary ammonium or phosphonium is constituted by a cation with formula:
R¹R²⁺N=CR³-R⁵-R³C=N⁺R¹R²
or
R¹R²⁺P=CR³-R⁵-R³C=P⁺R¹R²
where R¹, R² and R³, which may be identical or different, are defined as above and R₅ represents an alkylene or phenylene residue.

7. A process according to any one of claims 1 to 6, **characterised in that** the ammoniums and/or phosphoniums are selected from the group formed by N-butylpyridinium, N-ethylpyridinium, pyridinium, 3-ethyl-1-methyl-imidazolium, 3-butyl-1-methyl-imidazolium, diethylpyrazolium, N-butyl-N-methylpyrrolidinium, trimethylphenylammonium, tetrabutylphosphonium and tributyl-(tetradecyl)-phosphonium.

8. A process according to any one of claims 1 to 7, **characterised in that** the non-aqueous ionic solvent is selected from the group formed by N-butyl pyridinium hexafluorophosphate, N-ethylpyridinium tetrafluoroborate, pyridinium fluorosulphonate, 3-butyl-1-methyl imidazolium tetrafluoroborate, 3-butyl-1-methylimidazolium hexafluoroantimonate, 3-butyl-1-methyl-imidazolium hexafluorophosphate, 3-butyl-1-methyl-imidazolium trifluoroacetate, 3-butyl-1-methylimidazolium trifluoromethylsulphonate, trimethylphenylammonium hexafluorophosphate and tetrabutylphosphonium tetrafluoroborate.

9. A process according to any one of claims 1 to 8, **characterized in that** the cobalt and/or rhodium precursor compounds of the catalyst are selected from the group formed by cobalt and/or rhodium salts and carbonyl complexes.

10. A process according to any one of claims 1 to 9, **characterised in that** the cobalt and/or rhodium catalyst precursor compounds are selected from the group formed by acetylacetonates, carboxylates, dicobalt-octacarbonyl, cobalt-tetracarbonyl hydride, rhodium-dicarbonyl acetylacetonate and carbonyl clusters.

11. A process according to any one of claims 1 to 10, **characterised in that** the nitrogen-containing ligand is selected from the group formed by monoamines, di-, tri- and polyamines, imines, di-imines, pyridines, bipyridines, imidazoles, pyrroles and pyrazoles, all also containing in their formula at least one substituent carrying an ionic function (Q')⁺(A')⁻ such that at least the cation (Q')⁺ or anion (A')⁻ has the same chemical nature as cation Q⁺ or anion A⁻ of the non-aqueous ionic solvent defined above.

12. A process according to any one of claims 1 to 10, **characterised in that** the phosphorus-containing ligand is selected from the group formed by phosphines, polyphosphines, phosphine oxides and phosphites, all also containing in their formula at least one substituent carrying an ionic function (Q')⁺(A')⁻ such that at least the cation (Q')⁺ or anion (A')⁻ has the same chemical nature as cation Q⁺ or anion A⁻ of the non-aqueous ionic solvent defined above.

13. A process according to any one of claims 1 to 12, **characterised in that** the concentration of the cobalt and/or rhodium complex in the liquid ionic solvent is in the range 0.1 mmoles per litre to 5 moles per litre and the mole ratio between the nitrogen-containing ligand or the phosphorus-containing ligand and the cobalt and/or rhodium compound is in the range 0.1 to 500.

14. A process according to any one of claims 1 to 13, **characterised in that** at least one olefinically unsaturated compound selected from the group formed by mono-olefins, di-olefins, in particular conjugated di-olefins, olefinic compounds comprising one or more heteroatoms, in particular in unsaturated groups such as ketone and carboxylic acid functions, undergoes the hydroformylation reaction.

15. A process according to any one of claims 1 to 14, **characterised in that** the hydroformylation reaction is carried out with ratio of the partial pressures of hydrogen and carbon monoxide of 10:1 to 1:10, at a temperature in the range 30ºC to 200ºC and at a pressure in the range 1 MPa to 20 MPa.
